# EUROPÄISCHE PATENTSCHRIFT

(11) **EP 0 746 404 B1**
(45) Veröffentlichungstag und Bekanntmachung des Hinweises auf die Patenterteilung: **18.11.1998**
(21) Anmeldenummer: 95909760.1
(22) Anmeldetag: 21.02.1995
(51) Int. Cl.: B01D 61/44, C07C 277/06, C02F 1/469

(54) **VERFAHREN ZUR RÜCKGEWINNUNG VON GUANIDINSALZEN**
PROCESS FOR RECOVERING GUANIDINE SALTS
PROCEDE DE RECUPERATION DE SELS DE GUANIDINE

(30) Priorität: 22.02.1994 DE 4405546
(43) Veröffentlichungstag der Anmeldung: 11.12.1996
(73) Patentinhaber: NIGU CHEMIE GMBH, D-84478 Waldkraiburg (DE)
(72) Erfinder: KIESEWETTER, Erwin, D-52222 Stolberg (DE); STENGELE, Klaus-Peter, D-84539 Ampfing (DE)
(74) Vertreter: Hansen, Bernd, Dr. Dipl.-Chem.
(86) Internationale Anmeldenummer: EP9500629
(87) Internationale Veröffentlichungsnummer: WO9522398

(56) Entgegenhaltungen:
- FR-A- 2 198 705
- FR-A- 2 386 518
- US-A- 4 678 553
- GALVANOTECHNIK Bd. 7, 1991, EUGEN G. LENZE VERLAG, SAULGAU, Seiten 2298 - 2306 J. FRITSCH ET AL. 'Elektrodialyse und Diffusionsdialyse'

## Beschreibung

Die vorliegende Erfindung betrifft ein Verfahren zur Rückgewinnung von Guanidinsalzen aus verdünnten und verunreinigten wäßrigen Lösungen.

Guanidinsalze stellen wichtige Produkte bei organischen Syntheseverfahren sowie bei biotechnologischen Prozessen dar. Aus diesem Grund fallen bei den entsprechenden technischen Anwendungen große Mengen an Abwässern an, die neben Guanidinsalzen noch weitere zahlreiche Verunreinigungen organischer aber auch anorganischer Herkunft enthalten können. So sind entsprechende Guanidinsalzlösungen, wie sie bspw. in der Chromatographie, bei der De- bzw. Renaturierung von Proteinlösungen oder Auflösung von Inclusion-Bodies eingesetzt werden, in der Regel mit Restproteinen, reduzierenden Verbindungen (wie z. B. Sulfiden), nichtionischen Detergentien oder Puffersalzen verunreinigt. Außerdem ist die Konzentration der Guanidinsalze in diesen Lösungen sehr starken Schwankungen unterworfen.

In wirtschaftlicher Hinsicht aber auch aus Gründen des Umweltschutzes besteht ein großes Interesse, die betreffenden Guanidinsalze zu isolieren bzw. sie in möglichst reiner Form wieder zurückzugewinnen. Als einzige technische Möglichkeit bot es sich bisher an, die verdünnten Lösungen zuerst aufzukonzentrieren und anschließend die Guanidinsalze durch Kristallisation zu reinigen. Besonders nachteilig bei dieser Methode ist der hohe Energieaufwand, der zum Abdampfen der zum Teil großen Mengen an Wasser erforderlich ist. Außerdem bereitet die saubere Abtrennung der Verunreinigungen durch Kristallisation erhebliche Probleme, weshalb zur Rückgewinnung von einigermaßen reinen Produkten ein mehrfaches Umkristallisieren erforderlich ist und dieses Verfahren deshalb technisch ziemlich aufwendig erscheint.

Der vorliegenden Erfindung lag daher die Aufgabe zugrunde, ein Verfahren zur Rückgewinning von Guanidinsalzen aus wäßrigen Lösungen, enthaltend mindestens eine Verunreinigung ausgewählt aus Proteinen, Sulfiden, Puffersalzen, TRIS, EDTA und Eisen, zu entwickeln, welches die genannten Nachteile des Standes des Technik nicht aufweist, sondern es mit geringem technischen Aufwand ermöglicht, Guanidinsalze in hoher Reinheit aus diesen Lösungen zu isolieren.

Aus FR-A-2 386 518 ist bekannt, Guanidinsalze aus einem Produktstrom, die bei der Herstellung von Harnstoff aus Ammoniak und CO₂ erhalten werden, abzutrennen. Zwar wird neben einer Vielzahl anderer Methoden wie Adsorption an Ionenaustauscher, Umkehrosmose usw. auch allgemein die Elektrodialyse erwähnt, jedoch lassen sich keine Hinweise entnehmen, die Elektrodialyse zur Reinigung und Aufkonzentrierung aus verdünnten Lösungen mit den oben erwähnten Verunreinigungen einzusetzen.

Diese Aufgabe wurde erfindungsgemäß dadurch gelöst, daß man die entsprechende wäßrige Lösung (Diluat) einer Elektrodialyse unterwirft und das Guanidinsalz auf der Konzentratseite in aufkonzentrierter Form anreichert.

Eine detaillierte Übersicht zum Prinzip der Elektrodialyse und deren Anwendung findet sich bei J. Fritsch et al., "Elektrodialyse und Diffusionsdialyse", Galvanotechnik, Heft 7, 1991, Eugen G. Lenze Verlag, Saulgau.

Es hat sich nämlich überraschenderweise gezeigt, daß man auf diese Weise das entsprechende Guanidinsalz weitgehend von allen Verunreinigungen abtrennen und gleichzeitig (auf der Konzentratseite) ein Produkt in sehr hoher Konzentration gewinnen kann.

Aus der US-PS 4,678,553 ist zwar ein Verfahren zur Rückgewinnung von Polypeptiden und Proteinen aus Guanidinsalze enthaltenden Lösungen mit Hilfe der Elektrodialyse bekannt, doch liegen die Guanidinsalze hierbei in relativ konzentrierter Form vor und werden im Laufe der Elektrodialyse verdünnt.

Beim Verfahren entsprechend der vorliegenden Erfindung werden Guanidinsalzlösungen, die teilweise eine Konzentration von < 1 Gew.-% aufweisen, elektrodialysiert. Als Guanidinsalze können hierbei prakisch alle technisch wichtigen Salze eingesetzt werden wie z. B. Guanidinhydrochlorid, -sulfamat, -phosphat, -sulfat, -thiocyanat oder -nitrat.

Die Elektrodialyse selbst kann in den technisch üblichen Elektrodialyse-Zellen durchgeführt werden, die mit den bekannten ionenselektiven Membranen (im wesentlichen Ionenaustauschermembranen) ausgestattet sind. Im Hinblick auf die zu verwendende Elektrodenspülung hat es sich als besonders vorteilhaft erwiesen, auf Guanidinsalzlösungen zurückzugreifen wie z. B. Guanidinphosphat oder -sulfat. Auf diese Weise wird sichergestellt, daß in das Konzentrat keine unerwünschten Verunreinigungen miteingeschleust werden.

Die elektrischen Parameter wie Stromdichte und Spannung können entsprechend den technischen Möglichkeiten der Elektrodialyse-Zellen in gewissen Grenzen variiert werden. Technisch sinnvoll sind normalerweise 200 bis 1 000 A/m², insbesondere 300 bis 700 A/m² Elektrodenfläche, und Spannungen von ca. 0,5 bis 3 Volt pro Zellpaar.

Um einen einigermaßen befriedigenden Durchsatz erzielen zu können, werden in technisch üblicher Weise eine Vielzahl von Zellpaaren zu sogn. Membranstapeln zusammengefaßt, deren Anzahl in der Regel bei 5 bis 500, vorzugsweise 300 bis 400, Zellpaaren pro Stapel liegt.

In der Praxis wird das erfindungsgemäße Verfahren in der Weise durchgeführt, daß man die verdünnte und verunreinigte wäßrige Lösung (Diluat) kontinuierlich in die Zellpaare pumpt, die Elektrodialyse bei gewünschter Stromdichte und Spannung durchführt und das Konzentrat mit den angereicherten Guanidinsalzen kontinuierlich abzieht.

Ein besonderer Vorteil des erfindungsgemäßen Verfahrens besteht nun darin, daß die Elektrodialyse in einem sehr weiten pH-Bereich durchgeführt werden kann, der vorzugsweise zwischen 0 und 12 liegt und der nur von der Säure- oder Laugenbeständigkeit des eingesetzten Membranmaterials abhängig ist. Dies hat den großen Vorteil, daß man die eingesetzten Eluate normalerweise ohne große Vorbehandlung mit sauren oder alkalischen Zusätzen einsetzen kann.

Gemäß einer bevorzugten Ausführungsform der vorliegenden Erfindung wird die Elektrodialyse in zwei oder mehreren Stufen durchgeführt, wobei die betreffenden Membranstapel in Serie geschaltet werden, d. h. das Konzentrat aus dem ersten Membranstapel wird als Diluat im zweiten Membranstapel eingesetzt usw. Auf diese Weise ist eine weitere Aufkonzentrierung der betreffenden Eluate möglich. Während in der ersten Stufe das Eluat bspw. mit einer Konzentration < 1 Gew.-% bezogen auf das betreffende Guanidinsalz eingesetzt wird, besitzt das Konzentrat nach der ersten Stufe bereits eine Konzentration an Guanidinsalz von 10 bis 20 Gew.-%, nach der zweiten Stufe 20 bis 35 Gew.-%. Mit den derzeit zur Verfügung stehenden Elektrodialyse-Zellen ist eine Aufkonzentrierung der entsprechenden Guanidinsalzlösungen bis maximal 35 bis 50 Gew.-% möglich.

Diese aufkonzentrierten Lösungen weisen bereits eine sehr hohe Reinheit auf, die bei > 98 % liegt und die in der Regel nur geringe Mengen an anderen Salzen aufweisen. Falls erwünscht oder erforderlich, kann man an den Elektrodialyseschritt ohne weiteres eine Kristallisationsstufe anschließen, um das betreffende Salz in fester Form zu gewinnen.

Die besonderen Vorteile des erfindungsgemäßen Verfahrens bestehen im wesentlichen darin, daß man ausgehend von relativ stark verdünnten Lösungen mit stark schwankenden Verunreinigungen ohne großen technischen und energetischen Aufwand das Guanidinsalz bis zu einer Konzentration von 35 bis 50 Gew.-% aufkonzentrieren und gleichzeitig die in den Ausgangslösungen enthaltenen Verunreinigungen praktisch vollständig abtrennen kann.

Die nachfolgenden Beispiele sollen die Erfindung näher erläutern.

### Beispiele

### Beschreibung der Apparatur:

Die Durchführung der Beispiele erfolgte in einer Elektrodialyse-Vorrichtung, die gemäß den Patentansprüchen in der Weise modifiziert wurde, daß 2 Zellstapel, bestückt mit jeweils 7 kationenselektiven Membranen, 5 anionenselektiven Membranen und 11 Abstandshaltern (jeweils 5 Zellpaare), einem Membranabstand von 0,5 mm und einer effektiven Querschnittsfläche von 58 cm² in Serie geschalten wurden. Es ergaben sich somit 4 voneinander getrennte Kreisläufe:
- Diluat 1:: die Lösung, aus der das Salz zurückgewonnen werden soll
- Konzentrat 1:: eine intermediäre Lösung, in der sowohl eine erste Stufe der Aufkonzentrierung wie auch der Aufreinigung stattfindet
- Diluat 2:: ist gleich Konzentrat 1
- Konzentrat 2:: die Lösung, in der die zweite Stufe der Aufkonzentrierung stattfindet und das zurückgewonnene Salz durch einen Überlauf kontinuierlich entfernt wird
- Elektrodenspülung:: eine Lösung von chloridfreien Guanidinsalzen, die verhindern sollen, daß an der Anode eine Chlorgasentwicklung stattfindet

### Beispiel 1

30 kg einer Lösung von Guanidiniumchlorid aus der Herstellung eines rekombinanten Proteins (Diluat 1) werden in oben genannter Apparatur für 83 Stunden elektrodialysiert. In beiden Stromkreisen wird ein konstanter maximaler Wert der Stromstärke von 4 Ampere eingestellt, wobei die dafür notwendige Spannung automatisch entsprechend dem jeweiligen Widerstand des Zellstapels eingeregelt wird, jedoch auf ein Maximum von 12 Volt begrenzt wird. Das Produkt (Konzentrat 2) wurde in einem Überlaufbehälter gesammelt. Die Elektrodialyse wurde beendet, als Ausfällungen im Diluat 1 die Strömungsgeschwindigkeit auf unter 30 % des Anfangswertes herabsetzten.

### Anfangsbedingungen der Elektrodialyse:

- Diluat 1:: siehe unten
- Konzentrat 1 = Diluat 2:: 1 Gew.-% wässr. Lösung von Guanidiniumchlorid
- Konzentrat 2:: 1 Gew.-% wässr. Lösung von Guanidiniumchlorid
- Elektrodenspülung:: 8 Gew.-% wässr. Lösung von Guanidiniumdihydrogenphosphat

| Zustandparameter | Diluat 1 | Konzentrat 2 |
|---|---|---|
| Leitfähigkeit-Anfang [mScm⁻¹] | 163 | 10 |
| Leitfähigkeit-Ende [mScm⁻¹] | 105 | 190 |
| Guanidiniumchlorid-Anfang [Gew.-%] | ca. 23 | 1 |
| Guanidiniumchlorid-Ende [Gew.-%] | 9 | 46 |
| TRIS-Puffer-Anfang [mM] | ca. 25 | n.b. |
| TRIS-Puffer-Ende [mM] | n.b. | < 1 |
| Dithioerythritol-Ende [mM] | ca. 15 | nicht nachweisbar |
| Glutathion-Ende [mM] | ca. 15 | nicht nachweisbar |
| EDTA-Ende [mM] | ca. 7 | nicht bestimmt |
| Proteine-Ende [Gew.-%] | < 0,5 | nicht nachweisbar |
| Brij-35-Ende [Gew.-%] | < 0,5 | nicht nachweisbar |
| Eisen-Ende [mg/kg] | ca. 5 | < 0,5 |

Aus der Lösung aus Konzentrat 2 kann durch nachfolgende Kristallisation ein technisch reines Guanidiniumchlorid (Gehalt > 98,5 Gew.-%) gewonnen werden, das sich für den erneuten Einsatz in der Gewinnung rekombinanter Proteine eignet.

### Beispiel 2

25 kg einer Lösung von Guanidiniumchlorid aus der Herstellung eines rekombinanten Proteins (Diluat 1) werden in oben genannter Apparatur für 50 Stunden elektrodialysiert. In beiden Stromkreisen wird ein konstanter maximaler Wert der Stromstärke von 3 Ampere eingestellt, wobei die dafür notwendige Spannung automatisch entsprechend dem jeweiligen Widerstand des Zellstapels eingeregelt wird, jedoch auf ein Maximum von 12 Volt begrenzt wird. Das Produkt (Konzentrat 2) wurde in einem Überlaufbehälter gesammelt.

### Anfangsbedingungen der Elektrodialyse:

- Diluat 1:: siehe unten
- Konzentrat 1 = Diluat 2:: 1 Gew.-% wässr. Lösung von Guanidiniumchlorid
- Konzentrat 2:: 1 Gew.-% wässr. Lösung von Guanidiniumchlorid
- Elektrodenspülung:: 8 Gew.-% wässr. Lösung von Guanidiniumdihydrogenphosphat

| Zustandparameter | Diluat 1 | Konzentrat 2 |
|---|---|---|
| Leitfähigkeit-Anfang [mScm⁻¹] | 67 | 10 |
| Leitfähigkeit-Ende [mScm⁻¹] | 0,2 | 147 |
| Guanidiniumchlorid-Anfang [Gew.-%] | ca. 8 | 1 |
| Guanidiniumchlorid-Ende [Gew.-%] | < 0,5 | 42 |
| TRIS-Puffer-Anfang [mM] | ca. 25 | n.b. |
| TRIS-Puffer-Ende [mM] | n.b. | < 1 |
| Natriumacetat-Ende [Gew.-%] | 0,1 | 0,1 |
| Dithioerythritol-Ende [mM] | ca. 15 | nicht nachweisbar |
| Glutathion-Ende [mM] | ca. 15 | nicht nachweisbar |
| EDTA-Ende [mM] | ca. 5 | nicht bestimmt |
| Proteine-Ende [Gew.-%] | < 0,5 | nicht nachweisbar |
| Benzamidin Hydrochlorid [Gew.-%] | 0,05 | 0,05 |
| Eisen-Ende [mg/kg] | ca. 2 | < 0,5 |

Aus der Lösung aus Konzentrat 2 kann durch nachfolgende Kristallisation ein technisch reines Guanidiniumchlorid (Gehalt > 98,5 Gew.-%) gewonnen werden, das sich für den erneuten Einsatz in der Gewinnung rekombinanter Proteine eignet.

### Beispiel 3

2,5 kg einer Lösung von Guanidiniumthiocyanat, technisch reine Qualität, werden in oben genannter Apparatur für 43 Stunden elektrodialysiert. In beiden Stromkreisen wird ein konstanter maximaler Wert der Spannung von 6 Volt eingestellt, wobei die dafür notwendige Stromstärke automatisch entsprechend dem jeweiligen Widerstand des Zellstapels eingeregelt wird, jedoch auf ein Maximum von 3 Ampere begrenzt wird. Das Produkt (Konzentrat 2) wurde in einem Überlaufbehälter gesammelt.

### Anfangsbedingungen der Elektrodialyse:

- Diluat 1:: siehe unten
- Konzentrat 1 = Diluat 2:: 1 Gew.-% wässr. Lösung von Guanidiniumthiocyanat
- Konzentrat 2:: 1 Gew.-% wässr. Lösung von Guanidiniumthiocyanat
- Elektrodenspülung:: 8 Gew.-% wässr. Lösung von Guanidiniumdihydrogenphosphat

| Zustandparameter | Diluat 1 | Konzentrat 2 |
|---|---|---|
| Leitfähigkeit-Anfang [mScm⁻¹] | 70 | < 1 |
| Leitfähigkeit-Ende [mScm⁻¹] | 2 | 135 |
| Guanidiniumthiocyanat-Anfang [Gew.-%] | ca. 7 | 1 |
| Guanidiniumthiocyanat-Ende [Gew.-%] | < 0,3 | 17 |
| Eisen-Anfang [mg/kg] | ca. 5 | < 0,1 |
| Eisen-Ende [mg/kg] | ca. 2 | < 0,3 |

Aus der Lösung aus Konzentrat 2 kann durch nachfolgende Kristallisation ein technisch reines Guanidiniumthiocyanat (Gehalt > 99 Gew.-%) gewonnen werden, das sich für den erneuten Einsatz in der Gewinnung rekombinanter Proteine oder für die Molekularbiologie eignet.

### Beispiel 4

2,5 kg einer Lösung von Guanidiniumdihydrogenphosphat, technisch reine Qualität, werden in oben genannter Apparatur für 73 Stunden elektrodialysiert. In beiden Stromkreisen wird ein konstanter maximaler Wert der Spannung von 12 Volt eingestellt, wobei die dafür notwendige Stromstärke automatisch entsprechend dem jeweiligen Widerstand des Zellstapels eingeregelt wird, jedoch auf ein Maximum von 4 Ampere begrenzt wird. Das Produkt (Konzentrat 2) wurde in einem Überlaufbehälter gesammelt.

### Anfangsbedingungen der Elektrodialyse:

- Diluat 1:: siehe unten
- Konzentrat 1 = Diluat 2:: 0,1 Gew.-% wässr. Lösung von Guanidiniumdihydrogenphosphat
- Konzentrat 2:: 0,1 Gew.-% wässr. Lösung von Guanidiniumdihydrogenphosphat
- Elektrodenspülung:: 8 Gew.-% wässr. Lösung von Guanidiniumdihydrogenphosphat

| Zustandparameter | Diluat 1 | Konzentrat 2 |
|---|---|---|
| Leitfähigkeit-Anfang [mScm⁻¹] | 35 | 0,1 |
| Leitfähigkeit-Ende [mScm⁻¹] | 0,2 | 59 |
| Guanidiniumdihydrogenphosphat-Anfang [Gew.-%] | ca. 11 | 0,1 |
| Guanidiniumdihydrogenphosphat-Ende [Gew.-%] | < 0,3 | 23 |
| Eisen-Anfang [mg/kg] | ca. 3 | < 0,1 |
| Eisen-Ende [mg/kg] | ca. 1 | < 0,3 |

Aus der Lösung aus Konzentrat 2 kann durch nachfolgende Kristallisation ein technisch reines Guanidiniumdihydrogenphosphat (Gehalt > 99 Gew.-%) gewonnen werden, das sich für den üblichen Einsatz z. B. in der Herstellung von Flammschutzausrüstungen eignet.

### Beispiel 5

1065 kg einer Lösung von Guanidiniumchlorid aus der Herstellung eines rekombinanten Proteins (Diluat 1) werden in einer Apparatur wie in der Beschreibung der Patentansprüche mit 2 in Serie geschalteten Membranstapeln zu jeweils 25 Zellpaaren einer effektiven Querschnittsfläche von 0,16 m² und einem Membranabstand von 0,5 mm für 60 Stunden elektrodialysiert. In beiden Stromkreisen wird ein konstanter Anfangswert der Stromstärke von 60 Ampere eingestellt, wobei die dafür notwendige Spannung automatisch entsprechend dem jeweiligen Widerstand des Zellstapels eingeregelt wird, jedoch auf ein Maximum von 36 Volt begrenzt wird. Mit Abnahme der Leitfähigkeit im Diluat 1 Kreislauf werden die Stromstärken entsprechend der mit 36 Volt maximal erreichbaren Stromstärke im Diluat 1 Kreislauf in mehreren Schritten erniedrigt. Das Produkt (Konzentrat 2) wurde in einem Überlaufbehälter gesammelt. Die Elektrodialyse wurde durch übliche Spülzyklen unterbrochen, als Ausfällungen im Diluat 1 die Strömungsgeschwindigkeit auf unter 30 % des Anfangswertes herabsetzten und nach Freispülung wieder fortgesetzt.

### Anfangsbedingungen der Elektrodialyse:

- Diluat 1:: siehe unten
- Konzentrat 1 = Diluat 2:: 5 Gew.-% wässr. Lösung von Guanidiniumchlorid
- Konzentrat 2:: 5 Gew.-% wässr. Lösung von Guanidiniumchlorid
- Elektrodenspülung:: 8 Gew.-% wässr. Lösung von Guanidiniumdihydrogenphosphat

| Messprotokoll: | | | | | | |
|---|---|---|---|---|---|---|
| Zeit | Diluat 1 | | | Diluat 2 | Überlauf K-2 | |
| [h] | [kg] | [mScm⁻¹] | [Gew.-%] | [Gew.-%] | [kg] | [Gew.-%] |
| 0 | 1065 | 120 | 17,1 | - | 0 | - |
| 20,75 | 948 | 87 | 13,0 | 21,2 | 51,8 | 33,5 |
| 41,75 | 812 | 45 | 7,5 | 23,6 | 110,8 | 36,2 |
| 59,5 | 703 | 2 | 0,7 | 28,7 | 172,6 | 35,5 |

Die mittlere Stromausbeute betrug 57 %.

Aus der Lösung aus Konzentrat 2 kann durch nachfolgende Kristallisation ein technisch reines Guanidiniumchlorid (Gehalt > 98,5 Gew.-%) gewonnen werden, das sich für den erneuten Einsatz z. B. in der Gewinnung rekombinanter Proteine eignet.

## Patentansprüche

1. Verfahren zu Rückgewinnung von Guanidinsalzen aus wäßrigen Lösungen, enthaltend mindestens eine Verunreinigung ausgewählt aus Proteinen, Sulfiden, Puffersalzen, TRIS, EDTA und Eisen, dadurch gekennzeichnet, daß man die entsprechende wäßrige Lösung (Diluat) einer Elektrodialyse unterwirft und das Guanidinsalz auf der Konzentratseite in aufkonzentrierter Form anreichert.

2. Verfahren nach Anspruch 1, dadurch gekennzeichnet, daß das Guanidinsalz ausgewählt ist aus Guanidinhydrochlorid, -sulfamat, -phosphat, -sulfat, -thiocyanat und -nitrat.

3. Verfahren nach den Ansprüchen 1 oder 2, dadurch gekennzeichnet, daß man als Elektrodenspülung wäßrige Guanidinsalzlösungen, insbesondere Guanidinphosphatlösung, einsetzt.

4. Verfahren nach den Ansprüchen 1 bis 3, dadurch gekennzeichnet, daß die Stromdichte bei der Elektrodialyse 200 bis 1 000 A/m², vorzugsweise 300 bis 700 A/m² Elektrodenfläche, beträgt.

5. Verfahren nach den Ansprüchen 1 bis 4, dadurch gekennzeichnet, daß man die Spannung bei der Elektrodialyse pro Zellpaar auf 0,5 bis 3 Volt einstellt.

6. Verfahren nach den Ansprüchen 1 bis 5, dadurch gekennzeichnet, daß die Anzahl der Zellpaare pro Membranstapel 5 bis 500 beträgt.

7. Verfahren nach den Ansprüchen 1 bis 6, dadurch gekennzeichnet, daß während der Elektrodialyse der pH-Wert zwischen 0 und 12 liegt.

8. Verfahren nach den Ansprüchen 1 bis 7, dadurch gekennzeichnet, daß man 2 oder mehrere Membranstapel in Serie schaltet, in dem man das Konzentrat aus einem Membranstapel als Diluat im nachfolgenden Membranstapel einsetzt.

9. Verfahren nach den Ansprüchen 1 bis 8, dadurch gekennzeichnet, daß man die durch die Elektrodialyse gewonnene aufkonzentrierte Lösung des Guanidinsalzes anschließend noch einer Kristallisation unterwirft.

## Claims

1. Process for recovering guanidine salts from aqueous solutions containing at least one impurity selected from proteins, sulphides, buffer salts, TRIS, EDTA and iron, characterised in that the corresponding aqueous solution (diluate) is subjected to electrodialysis and the guanidine salt is enriched on the concentrate side in concentrated form.

2. Process according to claim 1, characterised in that the guanidine salt is selected from guanidine hydrochloride, guanidine sulphamate, guanidine phosphate, guanidine sulphate, guanidine thiocyanate and guanidine nitrate.

3. Process according to claims 1 or 2, characterised in that aqueous guanidine salt solutions, in particular guanidine phosphate solution, are used as the electrode rinse.

4. Process according to claims 1 to 3, characterised in that the current density during electrodialysis is 200 to 1,000 A/m², preferably 300 to 700 A/m², of electrode surface.

5. Process according to claims 1 to 4, characterised in that the voltage during electrodialysis is set at 0.5 to 3 volts per cell pair.

6. Process according to claims 1 to 5, characterised in that the number of cell pairs per membrane stack is 5 to 500.

7. Process according to claims 1 to 6, characterised in that during electrodialysis the pH value lies between 0 and 12.

8. Process according to claims 1 to 7, characterised in that 2 or more membrane stacks are connected in series by using the concentrate from one membrane stack as the diluate in the following membrane stack.

9. Process according to claims 1 to 8, characterised in the concentrated solution of the guanidine salt recovered by electrodialysis is then also subjected to crystallisation.

## Revendications

1. Procédé de récupération de sels de guadinine à partir de solutions aqueuses, contenant au moins une impureté, sélectionnée parmi des protéines, sulfures, sels tampons, TAIS, EDTA et fer, caractérisé en ce qu'on soumet la solution aqueuse (diluat) correspondante à une électrodialyse et on enrichit le sel de guadinine du côté du concentrat, sous une forme à concentration augmentée.

2. Procédé selon la revendication 1, caractérisé en ce que le sel de guadinine est sélectionné parmi l'hydrochlorure, le sulfamate, le phosphate, le sulfate, le thiocyanate et le nitrate de guadinine.

3. Procédé selon la revendication 1 ou 2, caractérisé en ce qu'on met en oeuvre, à titre de rinçage des électrodes, des solutions aqueuses de sels de guadinine, en particulier une solution de phosphate de guadinine.

4. Procédé selon les revendications 1 à 3, caractérisé en ce que la densité de courant lors de l'électrodialyse est de 200 à 1000 Ampères/m², de préférence 300 à 700 Ampères/m² de surface d'électrode.

5. Procédé selon les revendications 1 à 4, caractérisé en ce qu'on règle la tension lors de l'électrodialyse, par couple de cellules, à une valeur 0,5 à 3 Volt.

6. Procédé selon les revendications 1 à 5, caractérisée en ce que le nombre de couples de cellules par pile de membranes est de 5 à 500.

7. Procédé selon les revendications 1 à 6, caractérisé en ce que la valeur du pH est comprise entre 0 et 12 pendant l'électrodialyse.

8. Procédé selon les revendications 1 à 7, caractérisé en ce que l'on branche en série deux piles de membranes ou plus, en utilisant le concentrat issu d'une pile de membranes, à titre de diluat, dans la pile de membranes subséquente.

9. Procédé selon les revendications 1 à 8, caractérisé en ce qu'on soumet la solution du sel de guadinine à concentration augmentée, obtenue par l'électrodialyse, subséquemment encore à une cristallisation.
